# EUROPÄISCHE PATENTSCHRIFT

(11) **EP 1 373 245 B1**
(45) Veröffentlichungstag und Bekanntmachung des Hinweises auf die Patenterteilung: **27.12.2006**
(21) Anmeldenummer: 02722238.9
(22) Anmeldetag: 19.03.2002
(51) Int. Cl.: C07D 313/00, A01N 43/22

(54) **ZWISCHENVERBINDUNGEN ZUR HERSTELLUNG VON SPINOSYNEN**
INTERMEDIATES FOR PRODUCING SPINOSYNS
INTERMEDIAIRES DESTINES A LA FABRICATION DE SPINOSYNES

(30) Priorität: 29.03.2001 DE 10115407; 02.05.2001 DE 10121313
(43) Veröffentlichungstag der Anmeldung: 02.01.2004
(73) Patentinhaber: Bayer CropScience AG, 40789 Monheim (DE)
(72) Erfinder: STADLER, Marc, 42115 Wuppertal (DE); MAYER-BARTSCHMID, Anke, D 42489 Wulfrath (DE); SEIP, Stephan, 58332 Schwelm (DE); VELTEN, Robert, 51061 Köln (DE); JESCHKE, Peter, 51467 Bergisch Gladbach (DE); WEBER, Karlheinz, 42115 Wuppertal (DE)
(86) Internationale Anmeldenummer: PCT/EP2002/002999
(87) Internationale Veröffentlichungsnummer: WO 2002/079184

(56) Entgegenhaltungen:
- WO-A-97/00265
- US-A- 6 001 981
- J. MARTYNOW: "CHEMISTRY OF A8354A DERIVATIVES.1" JOURNAL OF ORGANIC CHEMISTRY., Bd. 59, 1994, Seiten 1548-60, XP002018758 AMERICAN CHEMICAL SOCIETY. EASTON., US ISSN: 0022-3263 in der Anmeldung erwähnt

## Beschreibung

Die vorliegende Erfindung betrifft Zwischenverbindungen zur Herstellung von Spinosynen, verschiedene Verfahren zu deren Herstellung, sowie die Verwendung dieser Zwischenverbindungen zur Herstellung von Spinosyn-Derivaten.

Bei den Spinosynen handelt es sich um bekannte Verbindungen. Spinosyne sind Fermentationsprodukte, die von Kulturen des Actinomyceten Saccharopolyspora spinosa hergestellt werden. Natürliche Spinosyne bestehen aus einem tetracyclischen Polyketidgrundgerüst (Aglykon) mit einem 12-gliedrigen Makrolidring und einem 5,6,5-cis-anti-trans-Tricyclus, sowie einem D-Forosamin- und einem 2,3,4-Tri-O-Methyl-L-Rhamnose-Zuckeranteil (Kirst et al. (1991), Tetrahedron Letters, 32:4839). Mehr als 20 verschiedene natürliche Spinosyne, der sogenannte A83543 Komplex, sind bisher beschrieben worden (vgl. WO 97/00265, WO 94/20518 und WO 93/09126). Diese Verbindungen variieren in der Substitution von einer oder einigen Methylgruppen am tetracyclischen Grundgerüst, am Forosamin- oder am Tri-Methyl-Rhamnose-Zuckeranteil. Ein 17-Pseudoaglykon, dem der Forosamin-Zuckeranteil fehlt, ist ebenfalls aus Kulturbrühen von S. spinosa isoliert worden.

Die Hauptkomponenten des von S. spinosa gebildeten A83543 Komplexes stellen die Varianten Spinosyn A und Spinosyn D dar, die die wesentlichen Bestandteile des Produktes Spinosad darstellen (vgl. Pesticide Manual, British Crop Protection Council, 11^{th} Ed., 1997, Seite 1272 und Dow Elanco trade maganzine Down to Earth, Vol. 52, NO:. 1, 1997 und die darin zitierte Literatur).

Wenn der Amino-Zucker nicht vorhanden ist, werden die Verbindungen als Spinosyn A, D, etc.-17-Pseudoaglykon bezeichnet; wenn der neutrale Zucker nicht vorhanden ist, werden die Verbindungen als Spinosyn A, D, etc.-9-Pseudoaglykon bezeichnet. Spinosyne ohne die beiden Zucker-Reste werden als Spinosyn-Aglykon bezeichnet.

Spinosyne sind geeignet zur Bekämpfung von Arachniden, Nematoden und Insekten, insbesondere von Lepidopteren und Dipteren. Man kann erwarten, dass Pflanzenschädlinge, die zur Zeit mit Spinosynen bekämpft werden, eine Resistenz gegen diese auf dem Markt befindlichen Wirkstoffe erzeugen können. Daher ist es wichtig, neue biologisch aktive Derivate von Spinosynen herzustellen, die derzeit zur Bekämpfung von Schädlingen verwendete Spinosyne ersetzen können. Synthetische Ansätze zur Herstellung von Spinosyn-Derivaten wurden von Martynow, J. G. und Kirst, H. A. in J. Org. Chem. 1994, 59, 1548 beschrieben. In dieser Publikation wird das 9,17-Diketon des Spinosyn-Aglykons sowie das 17-Keto-Derivat des Spinosyn-Aglykons erwähnt. Das Spinosyn-17-Pseudoaglykon und Spinosyn-9-Pseudoaglykon sind in WO 97/00265 und US-A 6 001 981 offenbart. Darin wird auch die Herstellung weiterer Derivate beschrieben, die durch semisynthetische Verfahren ausgehend von natürlichen Produkten erhältlich sind. Das Spinosyn-9-Pseudoaglykon, das an der Position C9 oxidiert ist, ist bekannt als insektizide Verbindung, die durch chemische Derivatisierung erzeugt wurde. Das 9-Keto-Spinosyn-Aglykon ist neu im Hinblick auf den Stand der Technik.

Es ist die Aufgabe der vorliegenden Erfindung, neue Zwischenverbindungen bereitzustellen, die für die Herstellung von Spinosyn-Derivaten geeignet sind.

Die Aufgabe wurde gelöst durch die Bereitstellung von Verbindungen der Formel (I) 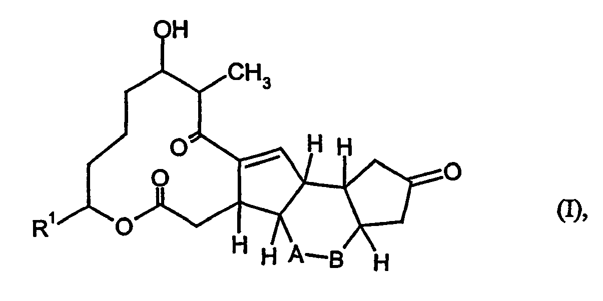 in welcher
- R¹: für Methyl oder Ethyl steht, und
- A-B: für eine der folgenden Gruppen steht: -HC=CH-, -HC=C(CH₃)-, -H₂C-CH₂-, -H₂C-CH(CH₃)-.
- R¹: steht bevorzugt für Ethyl.
- A-B: steht bevorzugt für die Gruppe -HC=CH-.

Die erfindungsgemäßen Verbindungen der Formel (I) können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen. Gegebenenfalls lassen sich die Isomere durch an sich bekannte Verfahren ineinander überführen.

Gegenstand der vorliegenden Erfindung sind auch chemische und biochemische/mikrobiologische Verfahren zum Herstellen der oben genannten Verbindungen der allgemeinen Formel (I).

Man erhält die Verbindungen der Formel (I), wenn man Verbindungen der Formel (II) 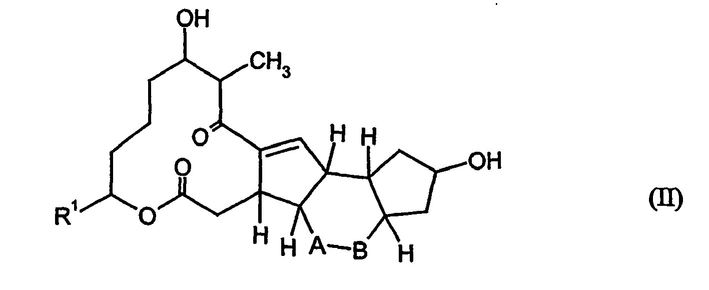 worin R¹ und A-B die oben angegebenen Bedeutungen haben,
mit einem Oxidationsmittel, gegebenenfalls in Gegenwart eines Verdünnungsmittels, umsetzt.

Das als Ausgangsverbindung für das erfindungsgemäße Verfahren verwendbare Spinosyn-Aglykon ist bekannt und kann nach dem in WO 01/16303 beschriebenen Verfahren hergestellt werden. In analoger Weise können die für das erfindungsgemäße Verfahren verwendbaren Ausgangsverbindungen ausgehend von den entsprechenden natürlichen Spinosynen erhalten werden.

Eine Vielzahl unterschiedlicher Oxidationsmittel ist für die Oxidation alkoholischer Gruppen bekannt (vgl. z. B. Oxidationsagenzien in: Organic Synthesis by Oxidation with Metal Compounds; Mijs, de Jonge; Plenum Verlag: New York, 1986; Manganese Compounds as Oxidizing Agents in Organic Chemistry; Arndt, Open Court Publishing Company: La Salle, IL, 1981; The Oxidation of Organic Compounds by Permanganate Ion and Hexavalent Chromium; Lee, Open Court Publishing Company: La Salle, IL, 1980). Danach kann eine Oxidation beispielsweise in Gegenwart von Permanganaten, wie Kaliumpermanganat, Halogenen, wie Chlor oder Brom, Metalloxiden, wie Mangandioxid oder Ruteniumtetroxid u. a. erfolgen.

Zahlreiche unterschiedliche Oxidationsmittel sind auch speziell nur für die Oxidation sekundärer Alkohole in der Literatur beschrieben, wie beispielsweise die Verwendung saurer Dichromate (vgl. Chromium Oxidations in Organic Chemistry; Cainelli, Cardillo, Springer Verlag: New York, 1984; Reagents for Organic Synthesis; Fieser, Vol. 1, Wiley: New York, 1967, S. 142-147, 1059-1064 und weitere Bd. aus dieser Reihe). Eine Lösung aus Chromsäure und Schwefelsäure in Wasser ist als Jones Reagenz (Bowden et al. (1946), J. Chem. Soc.: 39; Bowers et al. (1953), J. Chem. Soc.: 2548) bekannt. Drei andere Chrom (VI) Reagenzien (s. Mitteilung über eine vergleichende Studie des Jones's, Collins's und Corey's Reagenz in Warrener et al. (1978), Aust. J. Chem., 31: 1113) werden bekannterweise ebenfalls verwendet; beispielsweise Dipyridin-chrom(VI)-oxid (Collins's Reagenz) (vgl. z. B. Collins et al. (1968), Tetrahedron Lett.: 3363), Pyridinium-chlorchmmat (Corey's Reagenz) (vgl. Review: Luzzio and Guziec (1988), Org. Prep. Proced. Int., 20: 533-584) und Pyridinium-dichromat (vgl. Coates (1969), Corrigan Chem. Ind. (London): 1594; Corey, Schmidt (1979), Tetrahedron Lett.: 399). Für säureempfindliche Substrate ist beispielsweise auch der Einsatz von Chrom(VI)-oxid in Hexamethyl-phosphorsäuretriamid (HMPA) (vgl. Cardillo et al. (1976), Synthesis: 394) bekannt, ein Chrom(VI)-oxid-Pyridin-Komplex (vgl. Poos et al. (1953), J. Am. Chem. Soc., 75: 422) oder Trimethylsilylchromate (Moiseenkov et al. (1987), J. Org. Chem. USSR, 23: 1646) sind ebenfalls beschrieben. Natriumhypochlorit in Essigsäure ist für die Oxidation eines sekundären Alkohols in größerer Menge erwähnt (vgl. Stevens et al. (1980), J. Org. Chem., 45: 2030; Schneider et al. (1982), J. Org. Chem., 47: 364). Die Oxidationsreagenzien können aber auch polymergebunden vorliegen (vgl. Review: McKillop, Young (1979), Synthesis: 401-422). Sowohl Chromsäuren als auch Permanganate wurden auf diese Weise als Oxidationsmittel verwendet. Ebenfalls sind zahlreiche Phasen-Transfer-Reaktionen mit Permanganaten (vgl. Review: Lee, in Trahanovsky, Ref. 2, pt. D, S. 147-206), Chromsäuren (Hutchins et al. (1977), Tetrahedron Lett.: 4167; Landini et al. (1979), Synthesis: 134) und Rutenium-tetroxid (Morris, Kiely J. Org. Chem. (1987), 52: 1149) bekannt. Selbst Ultraschall-induzierte Oxidationsreaktionen sind denkbar - so ist die Verwendung von Kaliumpermanganat erwähnt (Yamawaki et al. (1983), Chem. Lett.: 379).

Darüber hinaus eignen sich die meisten Oxidationsmittel, die primäre Alkohole in Aldehyde oxidieren können, auch für die entsprechende Oxidation sekundärer Alkohole. Solche Oxidationsmittel für primäre Alkohole sind beispielsweise Pyridiniumdichromat, Tetrapropylammonium-perruthenat (Pr₄N⁺ RuO₄⁻), Cer-ammoniumnitrat (CAN), Silbercarbonat auf Celit (Fetizon et al. (1968), Acad. Sci., Ser. C, 267: 900), Na₂Cr₂O₇ in Wasser (Lee et al. (1970), J. Org. Chem., 35: 3589), Bleitetraacetat-Pyridin, Benzoylperoxid-Nickel-dibromid oder Dimethylsulfoxid in Gegenwart von Oxalylchlorid (Swern-Oxidation), Kupfer(II)-sulfat-Pentahydrat in Pyridin, Kupfer(II)-acetat in 70 % Essigsäure, Eisenchlorid in Wasser, Chrom(VI)-oxid in Eisessig oder Dichromiumtrioxid in Pyridin. Zu den Reagenzien, welche spezifisch eine sekundäre Hydroxygruppe auch in Gegenwart einer primären Hydroxygruppe oxidieren können, zählen beispielsweise Wasserstoffperoxid-Ammoniummolybdat (Trost et al. (1984), Isr. J. Chem., 24: 134), Natriumborat (NaBrO₃)-CAN (Tomioka et al., Tetrahedron Lett. 23: 539). N-Halogen-succinimide (Halogen = Chlor, Brom, Iod) können als Oxidationsmittel für Hydroxypruppen, auch in Gegenwart anderer oxidierbarer Gruppen eingesetzt werden (Variante nach Corey und Khim (1972), J. Am. Chem. Soc., 94:7586; Review: Filler (1963), Chem. Rev., 63: 21-43, S. 22-28). Beispielsweise eignet sich die Kombination von N-Iod-succinimid-Tetrabutylammoniumiodid zur Oxidation von sekundären Alkoholen in hohen Ausbeuten (Hanessian et al. (1981), Synthesis: 394).

Zu weiteren bekannten Oxidationsmethoden zählt auch die oxidative Dehydrierung, beispielsweise in Gegenwart von Katalysatoren wie Silber oder Kupfer-Katalysatoren (M. Muhler in: Handbook of Heterogenous Catalysis, VCH, Weinheim, 1997). Weitere milde katalytische oxidative Prozesse unter Verwendung von Platin- oder Palladium-Kohlenstoff Katalysatoren sind bekannt, die es erlauben, auch empfindliche Substanzklassen, beispielsweise Kohlenhydrate (M. Besson et al. (1995), J. Catal. 152: 116-122) oder Steroide (T. Akihisa et al. (1986), Bull. Chem. Soc. Jpn. 59: 680-685) zu oxidieren. Als effizienter kommerzieller Katalysator für die Oxidation ist beispielsweise der anorganische TS-1 Katalysator (oxide titanium silicalite), welcher in wässrigem Wasserstoffperoxid (30 % w/w) die katalytische Oxidation von primären und sekundären Alkoholen ermöglicht (R. Murugawel et al. (1997), Angew. Chem. Int. Ed. Engl., 36: 477-479), beschrieben.

Bevorzugt werden als Oxidationsmittel N-Halogensuccinimide, insbesondere N-Chlorsuccinimid, in Gegenwart von Dimethylsulfid (Swem-Oxidation), oder Pyridiniumdichromat eingesetzt.

Das erfindungsgemäße Verfahren zum Herstellen der neuen Verbindungen wird vorzugsweise unter Verwendung von Verdünnungsmitteln durchgeführt. Verdünnungsmittel werden vorzugsweise in einer solchen Menge eingesetzt, dass das Reaktionsgemisch während des ganzen Verfahrens gut rührbar bleibt.

In Abhängigkeit des zuvor genannten Oxidationsmittels kommen als Verdünnungsmittel neben Wasser oder wässrigem Wasserstoffperoxid auch saure Verdünnungsmittel, wie beispielsweise konzentrierte oder partiell verdünnte Essigsäure, und basische Verdünnungsmittel, wie beispielsweise Pyridin, in Betracht.

Weiterhin kommen praktisch alle inerten organischen Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Anisol, Chlorbenzol, Dichlorbenzol, Petrolether, Hexan, Cyclohexan, Dichlormethan, 1,2-Dichlorethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Sulfolan.

Selbstverständlich kann man das erfindungsgemäße Verfahren auch in Gemischen der genannten Lösungsmittel durchführen.

Besonders bevorzugt wird als Verdünnungsmittel Dichlormethan eingesetzt.

Weiterhin wird vorzugsweise die Reaktion gemäß dem erfindungsgemäßen Verfahren unter Inertgas durchgeführt.

Die Reaktionstemperaturen können bei dem erfindungsgemäßen Verfahren in einem größeren Bereich variiert werden. Im Allgemeinen arbeitet man bei Temperaturen zwischen - 100°C und + 150°C, vorzugsweise bei Temperaturen zwischen - 20°C und + 50°C.

Das erfindungsgemäße Verfahren wird im Allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsverbindungen im Allgemeinen in annähernd äquimolaren Mengen eingesetzt. Es ist jedoch auch möglich, das Oxidationsmittel im Unterschuss zu verwenden.

Die Aufarbeitung erfolgt nach üblichen Methoden.

Die neuen Verbindungen der allgemeinen Formel (I) können auch durch Biokonversion ausgehend von Verbindungen der allgemeinen Formel (II) hergestellt werden.

Selektive und/oder stereospezifische Oxidationen von Hydroxyl-Gruppen natürlicher Produkte und synthetischer Verbindungen durch Biokonversion unter Verwendung von Mikroorganismen oder deren Enzyme sind in der Literatur beschrieben. Insbesondere wurden Zellen und/oder Enzyme von Actinomyceten (Nocardia, Streptomyces) und anderen Gram-positiven (Bacillus, Clostridium) oder Gram-negativen Bakterien für die spezifische Oxidation von Steroid-Verbindungen, deren chemische Zugänglichkeit begrenzt ist, verwendet. Beispiele, die insbesondere Enzym-Klassen wie Hydroxysteroid-Dehydrogenasen oder Cholesterin-Oxidasen betreffen, sind in der folgenden Tabelle aufgelistet:

| **Organismus/Enzym** | **Klasse der chemischen Verbindung** | **Referenz** |
|---|---|---|
| Multi-Enzymsystem Clostridium absonum/Bacillus megaterium/Proteus; 7-alpha-Hydroxysteroid- Dehydrogenase (HSDH) + 12-alpha-HSDH und andere Enzyme | D-Ketoursocholinsäure (Steroid) | Bovara, R. et al. (1996): Biotechnology Lett. 19,305-308 |
| Bacillus stearothermophilus Zellen | bizyklische Derivate der Oktenonsäure | Giovannini et al. (1996): Tetrahedron 52,1669-1676 |
| Streptomyces sp. Cholesterin-Oxidase | *delta*-4-Cholestenon (Steroid) | Lee & Bielmann (1988): Tetrahedron 44, 1135-1139 |
| Multi-Enzymsystem Clostridium absonum/Proteus; 3-alpha-Hydroxysteroid- Dehydrogenase und andere Enzyme | Cholinsäurederivate (Steroide) | Riva, S. et al. (1986): J. Org Chem. 51, 2902-2906 |
| E. coli; 7-alpha-Hydroxysteroid-Dehydrogenase (HSDH) | Cholinsäurederivate (Steroide) | Bovara et al. (1993): J. Org. Chem. 58, 499-501 |
| Brevibacterium sp. Cholesterin-Oxidase | 7-*beta*-Hydroxytestosteron (Steroid) | Labaree et al. (1997): Steroids 62, 482-486 |
| Nocardia rhodochrous; Zellen und Cholesterin-Oxidase | Diosgenon (Steroid) | Saunders et al. (1986) Enzyme Microb. Technol. 9, 549-555 |

Erfindungsgemäß kann man Verbindungen der Formel (II) 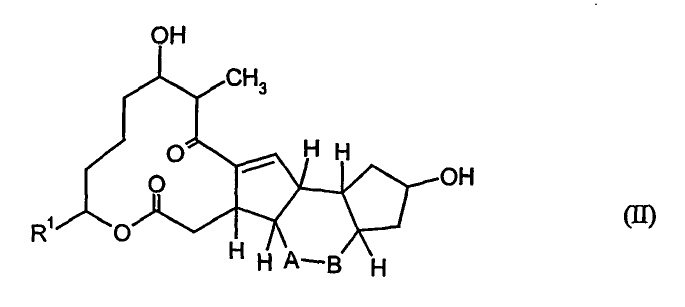 worin R¹ und A-B die oben angegebenen Bedeutungen haben,
mit einem Mikroorganismus in einem wässrigen Nährmedium unter aeroben Bedingungen in Kontakt bringen und danach Verbindungen der Formel (I) isolieren.

Anstelle der Mikroorganismen können auch Enzymextrakte und aufgereinigte Enzyme, gegebenenfalls nach Zugabe oder unter Regenerierung der benötigten Cofaktoren, verwendet werden, die nach üblichen Verfahren ausgehend von diesen Mikroorganismen erhältlich sind.

Vorzugsweise wird für das erfindungsgemäße Verfahren ein Mikroorganismus aus der Gattung Bacillus oder aus der Gruppe der Actinomyceten, insbesondere aus der Gattung Streptomyces, oder ein Pilz, insbesondere aus der Klasse der Zygomyceten, und hier bevorzugt der Gattungen Zygorhynchus oder Mucor - bzw. ausgehend davon hergestellte Enzymextrakte oder aufgereinigte Enzyme - verwendet.

Besonders bevorzugt wird für das erfindungsgemäße Verfahren ein Stamm aus der Art Bacillus simplex, Bacillus megaterium, Streptomyces argillaceus, Streptomyces scabies, Streptomyces mirabilis, Streptomyces pseudovenecuelae, Zygorhynchus moelleri oder Mucor circinelloides eingesetzt.

Ganz besonders bevorzugt wird für das erfindungsgemäße Verfahren ein Stamm mit den kennzeichnenden Merkmalen der folgenden Stämme verwendet:

| **Name** | **Hinterlegungs-Nr.** |
|---|---|
| Streptomyces argillaceus | DSM 14030 |
| Streptomyces scabies | DSM 14029 |
| Bacillus megaterium | DSM 333 |
| Bacillus megaterium | DSM 339 |
| Bacillus simplex | DSM 14028 |
| Streptomyces spec. | DSM 14077 |
| Streptomyces mirabilis | DSM 14078 |
| Streptomyces pseudovenecuelae | DSM 14079 |
| Zygorhynchus moelleri | DSM 14198 |
| Mucor circinelloides | DSM 14199 |

Die in der Tabelle genannten Stämme sind bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt worden.

Die Stämme sind in Beispiel 9 näher beschrieben. Es können nicht nur die hinterlegten Stämme als solche, sondern auch Mutanten davon verwendet werden, solange diese Mutanten die kennzeichnenden Merkmalen der hinterlegten Stämme besitzen. Dies bedeutet, dass die Mutanten noch die Fähigkeit besitzen müssen, die erfindungsgemäße Biokonversion durchführen zu können.

Vorzugsweise enthält das wässrige Nährmedium eine assimilierbare Kohlenstoffquelle und eine assimilierbare Stickstoffquelle.

Die Verbindungen der Formel (I) werden beispielsweise produziert, wenn ein Stamm aus den in der Tabelle erwähnten Arten in einem wässrigen Nährmedium unter aeroben Bedingungen in der Gegenwart von Verbindungen der Formel (II) fermentiert werden. Typischerweise werden die Mikroorganismen in einem Nährmedium, welches eine Kohlenstoffquelle und gegebenenfalls ein proteinartiges Material enthält, fermentiert. Bevorzugte Kohlenstoffquellen umfassen Glucose, braunen Zucker, Saccharose, Glycerin, Stärke, Maisstärke, Lactose, Dextrin, Melasse etc. Bevorzugte Stickstoffquellen umfassen Baumwollsaatmehl, Hefe, autolysierte Bäckerhefe, feste Milchbestandteile, Sojabohnenmehl, Maismehl, pankreatische oder papainisch verdaute Spaltprodukte von Kasein, feste Destillationsbestandteile, Brühen aus tierischem Pepton, Fleisch- und Knochenstücke etc. Vorzugsweise werden Kombinationen dieser Kohlenstoff- und Stickstoffquellen verwendet. Spurenelemente, wie beispielsweise Zink, Magnesium, Mangan, Kobalt, Eisen etc. müssen dem Fermentationsmedium nicht zugefügt werden, solange Leitungswasser und nicht-gereinigte Bestandteile als Mediumkomponenten verwendet werden.

Die Herstellung der Verbindungen der allgemeinen Formel (I) kann bei jeder Temperatur induziert werden, die ein ausreichendes Wachstum der Mikroorganismen gewährleistet. Vorzugsweise beträgt die Temperatur zwischen 21°C und 32°C, besonders bevorzugt ungefähr 28°C.

Im Allgemeinen wird eine optimale Produktion der Verbindungen der Formel (I) innerhalb von 1 bis 10 Tagen nach Zugabe der Verbindungen der Formel (II) zu der Kultur erhalten, vorzugsweise in ungefähr 2 bis 6 Tagen. Die Fermentationsbrühe bleibt normalerweise während der Fermentation schwach basisch (pH 7,4 bis pH 8,0). Der End-pH ist teilweise abhängig von dem gegebenenfalls verwendeten Puffer und teilweise vom anfänglichen pH des Kulturmediums. Vorzugsweise wird der pH auf ungefähr 6,5 bis 7,5 vor der Sterilisierung eingestellt, besonders bevorzugt auf pH 7,2.

Die Produktion der erfindungsgemäßen Verbindungen findet sowohl in Schüttelflaschen als auch in Rühr-Fermentern statt. Wenn die Kultivierung in Schüttelflaschen oder großen Kesseln und Tanks durchgeführt wird, wird vorzugsweise die vegetative Form anstatt der Sporen-Form der Mikroorganismen zum Animpfen verwendet, um eine deutliche lag-Phase bei der Produktion der Metabolite und damit eine ineffiziente Ausnutzung der Gerätschaften zu vermeiden. Demnach ist es vorteilhaft, ein vegetatives Inokulum in einem wässrigen Nährmedium durch Animpfen dieses Mediums mit einem Aliquot einer Boden- oder Schrägröhrchen-Kultur herzustellen. Nachdem ein frisches, aktives, vegetatives Inokulum auf diese Weise vorbereitet worden ist, wird dieses aseptisch in weitere Schüttelflaschen oder weitere geeignete Gerätschaften für die Fermentation der Mikroorganismen überführt. Das Medium, in welchem das vegetative Inokulum hergestellt wird, kann identisch oder unterschiedlich zu demjenigen Medium sein, das für die Herstellung der erfindungsgemäßen Verbindungen verwendet wird, solange es ein ausreichendes Mikroorganismenwachstum gewährleistet.

Im Allgemeinen wird die Herstellung der erfindungsgemäßen Verbindungen unter Verwendung der oben genannten Mikroorganismen unter aeroben Bedingungen in Rühr-Fermentem bewerkstelligt. Die Herstellung ist jedoch unabhängig von den verwendeten Fermentern und Starterkulturen. Die erfindungsgemäßen Verbindungen können auch über Schüttelkulturen erhalten werden. Für großvolumige Fermentationen wird vorzugsweise ein vegetatives Inokulum verwendet. Das vegetative Inokulum wird durch Animpfen eines kleinen Volumens des Kulturmediums mit der Sporenform, Myzelfragmenten oder einem lyophilisierten Pellet des Mikroorganismus hergestellt. Das vegetative Inokulum wird dann in einen Fermentationskessel überführt, worin nach einer geeigneten Inkubationszeit in Gegenwart der Verbindungen der allgemeinen Formel (II) die erfindungsgemäßen Verbindungen in einer optimalen Ausbeute produziert werden. Gewöhnlich wird bei einem aeroben Submers-Fermentationsverfahren sterile Luft durch das Kulturmedium geleitet. Für ein effizientes Wachstum der Mikroorganismen liegt das verwendete Luftvolumen im Bereich von ungefähr 0,25 bis ungefähr 0,5 Volumen Luft pro Volumen Kulturmedium pro Minute (vvm). Ein optimales Verhältnis in einem 101 Kessel ist ungefähr 0,3 vvm mit Bewegung, die durch einen konventionellen mit ungefähr 200- 500 U/min, vorzugsweise mit 300 U/min rotierenden Propeller erzeugt wird. Die Zugabe einer kleinen Menge, wie beispielsweise 1 ml/l, eines schaumverhindernden Mittels, wie Silikon zum Fermentationsmedium ist notwendig, falls die Schaumbildung ein Problem darstellt.

Bevorzugte Fermentationsbedingungen und Medien sind in den Beispielen beschrieben.

Die Biotransformation der Verbindungen der Formel (II) zu den erfindungsgemäßen Verbindungen beginnt im Allgemeinen nach ungefähr 48 Stunden und findet für mindestens 6 Tage während der Fermentationsperiode statt. Die Peak-Produktion wird zwischen ungefähr 5 bis 7 Tagen Fermentationszeit erreicht, bei der Verwendung von Bacillus-Stämmen bereits nach 3-4 Tagen.

Die erfindungsgemäßen Verbindungen als Biotransformationsprodukt können aus dem Fermentationsmedium durch übliche Verfahren isoliert werden.

Die erfindungsgemäßen Verfahren sind vor allem in der Biomasse der fermentierten Mikroorganismen vorhanden, sie können jedoch auch in geringen Mengen im Kulturfiltrat der Fermentationsbrühe vorkommen. Die Kulturbrühe kann auf einfache Weise durch Filtrieren durch eine Filterpresse abgetrennt werden.

Verschiedene Verfahren können verwendet werden, um die erfindungsgemäßen Verbindungen aus der Fermentationsbrühe zu isolieren und zu reinigen, wie z.B. durch chromatographische Adsorptionsverfahren (beispielsweise durch Säulenchromatographie, Flüssig-Flüssig-Verteilungschromatographie, Gelpermeationschromatographie) gefolgt von Elution mit einem geeigneten Lösungsmittel, und Kristallisation aus Lösungsmitteln, sowie Kombinationen davon. In einem bevorzugten Reinigungsverfahren werden die erfindungsgemäßen Verbindungen aus der Biomasse, aus den Myzelien oder aus Extrakten des Überstands extrahiert. Letztere können unter Verwendung von Adsorptionsharzen, wie beispielsweise XAD, HP20 oder Lewapol hergestellt werden. Säulenchromatographie-Methoden, vorzugsweise über Kieselgel oder modifizierte Kieselgele werden verwendet, um eine Anfangs-Reinigung durchzuführen. Die endgültige Reinigung der erfindungsgemäßen Verbindungen wird vorzugsweise durch präparative Hochleistungs-Flüssigchromatographie (HPLC) erreicht.

Die erfindungsgemäßen Verbindungen können zur Herstellung von biologisch aktiven, insbesondere insektiziden, Spinosyn-Derivaten verwendet werden.

Verwendet man beispielsweise zur Glykosidierung der erfindungsgemäßen Verbindung der Formel (Ia) ein aktiviertes Forosamin (D-Forosamin: vgl. EP-A 0 375 316) als Aminozucker der Formel (III), worin LG für eine bekannte, geeignete Abgangsgruppe (LG = leaving group), wie beispielsweise Brom oder 2,2,2-Trichlorethanimidat (= Trichloracetimidat) stehen kann, so erhält man das aus WO 97/00265 und US-A 6 001981 bekannte 9-Keto-Spinosyn A-9-Pseudoaglykon der Formel (IVa) (vgl. Schema 1).

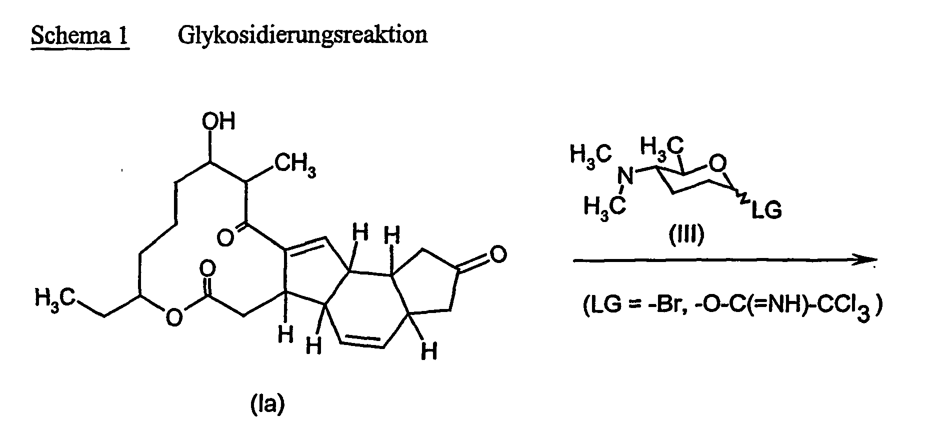

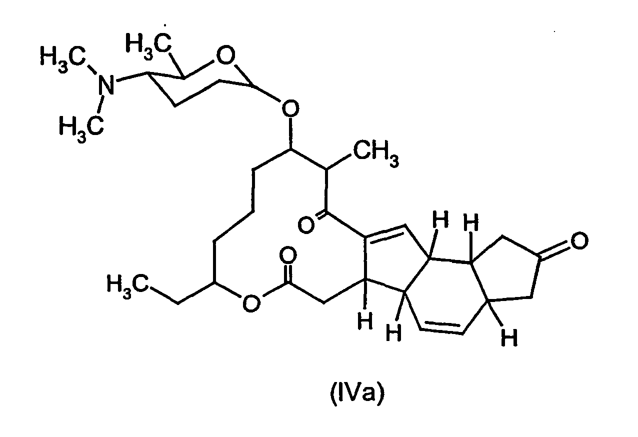

Die Darstellung eines aktivierten Forosamins als Aminozucker der Formel (III), beispielsweise des α-D-Forosaminylbromid Hydrobromids bzw. des α-L-N-Fmoc-Forosaminylbromids (LG = Brom), und dessen Glykosidierungsreaktion ist aus WO 97/00265 und US-A 6 001 981 bzw. D. A. Evans et al. (1993), J. Am. Chem. Soc. 115: 4497-4513 bekannt. Ferner ist die Darstellung eines aktivierten Aminozuckers der Formel (III), beispielsweise des 1-(2,2,2-Trichlorethanimidat)-4,6-didesoxy-4-(dimethylamino)-5-C-methyl-β-D-ribo-hexopyranose-2,3-diacetats (LG = 2,2,2-Trichlorethanimidat), und dessen stereoselektive Glykosidierungsreaktion bekannt (vgl. I. Sato et al. (1999), Chem. Lett. 9: 867-868; vgl. zusätzlich auch die Verwendung von Trichloracetimidaten bei der Herstellung von Glucospingolipiden: G. R. Duffin et al. (2000), J. Chem. Soc., Perkin Trans. 1: 2237-2242).

Die biologische Wirksamkeit des 9-Keto-Spinosyn A-9-Pseudoaglykons der Formel (IVa) gegen Stomoxys calcitrans (stable fly) und Phormia regina (blow fly) ist bereits in WO 97/00265 und US-A 6 001 981 beschrieben.

Des Weiteren sind aus WO 97/00265 und US-A 6 001 981 zwei Folgereaktionen (Weg a und b) mit dem 9-Keto-Spinosyn A-9-Pseudoaglykon der Formel (IVa) bekannt, in denen die Reaktivität der Carbonylgruppe in 9-Position genutzt wird (vgl. Schema 2).

Beispielsweise ist es möglich, das 9-Keto-Spinosyn A-9-Pseudoaglykon der Formel (IVa) mit dem Grignard-Reagenz Methylmagnesiumchlorid (V) zu einem chromatographisch auftrennbaren Gemisch von (9S)- und (9R)-9-Methyl-Spinosyn A-9-Pseudoaglykon der Formel (VIa) und (VIb) umzusetzen (Weg a). In einem weiteren bekannten Ausführungsbeispiel wird die Reaktion des 9-Keto-Spinosyn A-9-Pseudoaglykons der Formel (IVa) mit Morpholin (VII) in Gegenwart von Natriumcyanoborhydrid unter Bildung des 9-Desoxy-9-(N-morpholinyl)-Spinosyn A-9-Pseudoaglykons der Formel (VIII) erläutert (vgl. Schema 2, Weg b). 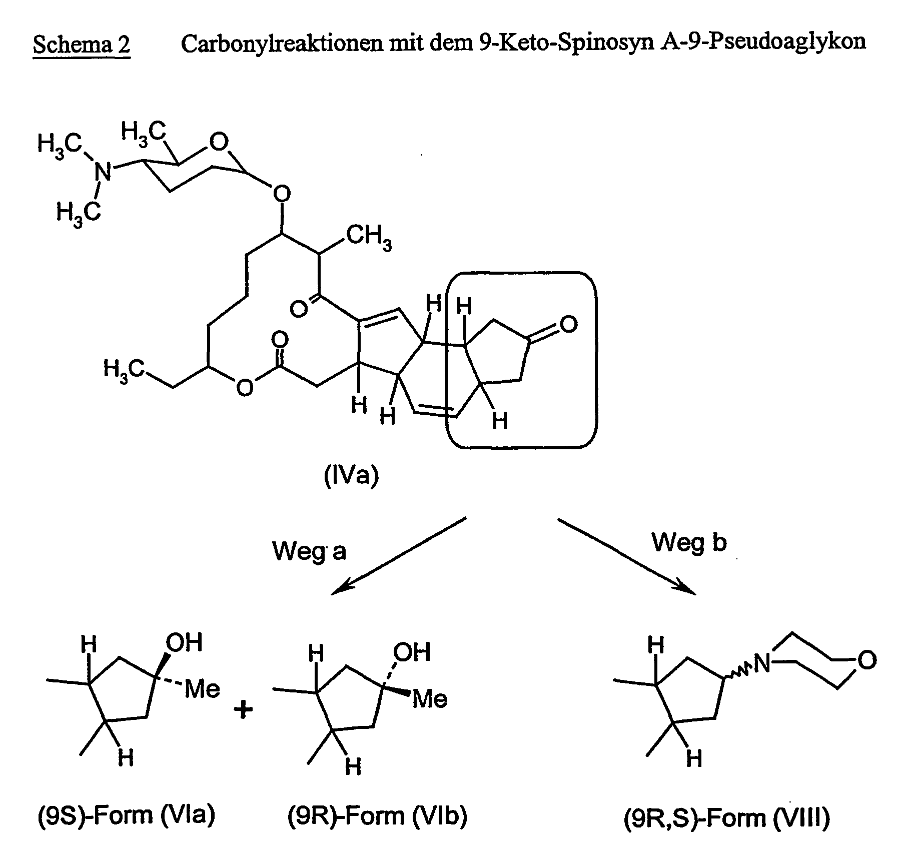
- Weg a:: MeMgCl (V), Tetrahydrofuran
- Weg b:: Morpholin (VII), Methanol, Natriumcyanoborhydrid

Für das (9S)- und (9R)-9-Methyl-Spinosyn A-9-Pseudoaglykon der Formel (VIa) und (VIb) ist eine biologische, insbesondere insektizide, Wirksamkeit gegen Aphis gossipii (cotton aphid), Heliothis virescens (tobacco budworm) und Stomoxys calcitrans (stable fly) bereits in WO 97/00265 und US-A 6 001 981 beschrieben. Auch für das 9-Desoxy-9-(N-morpholinyl)-Spinosyn A-9-Pseudoaglykon der Formel (VIII) wird eine entsprechende biologische, insbesondere insektizide, Wirksamkeit gegen Stomoxys calcitrans (stable fly) in WO 97/00265 und US-A 6 001981 gezeigt.

### Beispiele

### Beispiel 1

### Herstellung des Spinosyn-Aglykons aus Tracer®

Die Verbindung der Formel (II), worin R¹ für Ethyl und A-B für die Gruppe -HC=CH- steht (im Folgenden Verbindung (2) genannt), wurde wie in WO 01/16303 beschrieben hergestellt.

### Beispiel 1a)

### Herstellung des 5,6-Dihydrospinosyn-Aglykons aus Tracer®

Die Verbindung der Formel (II), worin R¹ für Ethyl und A-B für die Gruppe - H₂C-CH₂- steht (5,6-Dihydrospinosyn-Aglykon, im Folgenden Verbindung (4) genannt), wurde analog zu WO 01/16303 aus 5,6-Dihydrospinosyn A hergestellt. Die Darstellung von 5,6-Dihydrospinosyn A aus Spinosyn A ist aus WO 97/00265 und US-A 6 001 981 bekannt. Spinosyn A seinerseits wurde wie in WO 01/16303 beschrieben aus Tracer® hergestellt.

### Beispiel 2

### Verwendete Stämme

Tabelle: Stämme, die zur Biotransformation der Verbindung (2) zu dem entsprechenden 9-Keto-Derivat (im Folgenden Verbindung (1) genannt) befähigt sind:

| **Name** | **Interne Bezeichnung** | **Hinterlegungs-Nr.** |
|---|---|---|
| Streptomyces argillaceus | ATCC 12956 | DSM 14030 |
| Streptomyces scabies | BS 2134 | DSM 14029 |
| Bacillus megaterium | DSM 333 | DSM 333 |
| Bacillus megaterium | DSM 339 | DSM 339 |
| Bacillus simplex | DSM 1318 | DSM14028 |
| Streptomyces spec. | BA 312 | DSM 14077 |
| Streptomyces mirabilis | BA 579 | DSM14078 |
| Streptomyces pseudovenecuelae | WS 2199 | DSM 14079 |
| Zygorhynchus moelleri | WP0796 | DSM 14198 |
| Mucor circinelloides | WP0799 | DSM 14199 |

| | | |
|---|---|---|
| ATCC= American Type Culture Collection, Rockville, MD. DSM= Deutsche Sammlung von Mikroorganismen und Zellkulturen, Braunschweig, Deutschland. | | |

### Beispiel 3

### Biotransformation mit Streptomyces argillaceus DSM 14030, Streptomyces scabies DSM 14029, Streptomyces spec. DSM 14077, Streptomyces mirabilis DSM 14078, Streptomyces pseudovenecuelae DSM 14079, Zygorhynchus moelleri DSM 14198 oder Mucor circinelloides DSM 14 199

Beispielhaftes Protokoll der Biotransformation für die Herstellung der Verbindung (1) aus der Verbindung (2).

### Beispiel 3a)

### Herstellung der Vorkulturen von Streptomyces argillaceus DSM 14030, Streptomyces scabies DSM 14029, Streptomyces spec. DSM 14077, Streptomyces mirabilis DSM 14078, Streptomyces pseudovenecuelae DSM 14079, Zygorhynchus moelleri DSM 14198 oder Mucor circinelloides DSM 14199

Zur Herstellung der Vorkulturen wurden die Stämme Streptomyces argillaceus DSM 14030, Streptomyces scabies DSM 14029, Streptomyces spec. DSM 14077, Streptomyces mirabilis DSM 14078, Streptomyces pseudovenecuelae DSM 14079, Zygorhynchus moelleri DSM 14198 oder Mucor circinelloides DSM 14199 in folgenden Medien angezogen: Medium 1: Hefe-Malz Medium: D-Glucose 0.4 %, Hefeextrakt 0.4 %, Malzextrakt 1.0 %, ad 1 Liter Leitungswasser (pH mit wässriger HCl auf 6,5 bei den Pilzen und mit wässriger NaOH auf 7,2 bei den Bakterien eingestellt) oder TSB Medium (Medium 2): Trypticase Soy Broth (Difco) (30 g/l), ad 1 Liter Leitungswasser. Der pH-Wert des Mediums wurde mit wässriger NaOH auf 7.2 eingestellt. Die Medien wurden jeweils bei 121°C und 1,1 bar Überdruck für 20 Minuten sterilisiert.

Je 2 ml einer Myzelsuspension in 50 % Glyzerin wurden als Inokulum für 2 x 150 ml Medium 1 in einem 1000 ml Erlenmeyerkolben angesetzt und 72 Stunden bei 240 Umdrehungen pro Minute (U/min) auf einer Rundschüttelmaschine inkubiert. Diese Kulturen wurden entweder benutzt, um neue Glyzerinkonserven herzustellen, die bei -20°C aufbewahrt wurden, oder sie dienten als Inokulum für die Produktionskulturen (siehe 3b).

### Beispiel 3b)

### Herstellung der Produktionskulturen von Streptomyces argillaceus DSM 14030, Streptomyces scabies DSM 14029, Streptomyces spec. DSM 14077, Streptomyces mirabilis DSM 14078, Streptomyces pseudovenecuelae DSM 14079, Zygorhynchus moelleri WP0796 oder Mucor circinelloides DSM 14199

Zur Herstellung der Produktionskulturen wurden je 2 ml einer wie unter 3a) beschriebenen Vorkultur als Inokulum für 100 x 150 ml Medium GS (Glukose (20 g/l), Sojamehl (20 g/l), Stärke (20 g/l), NaCl (2,5 g/l), CaCO₃ (5 g/l), MgSO₄ x 6 H₂O (0,5 g/l), KH₂PO₄ (0,25 g/l) eingesetzt. Vor dem Beimpfen wurde der pH-Wert mit KOH auf 6.8 eingestellt und es wurde bei 121°C und 1,1 bar Überdruck für 20 Minuten sterilisiert. Die Verbindung (2) wurde in einer Endkonzentration von 50 - 500 mg/l Medium in methanolischer Lösung (100 mg/ml Methanol) wahlweise zu Beginn der Fermentation oder während der Fermentation zu einem beliebigen Zeitpunkt bis hin zu einer Fermentationsdauer von 160 Stunden zugesetzt. Die Biotransformation wurde nach 240 Stunden abgebrochen. Zur Verfolgung des Biotransfonnations-Prozesses dienten tägliche Proben von 50 ml, die steril entnommen und mit Hilfe der analytischen HPLC untersucht wurden. Unter diesen Bedingungen wurden, vorzugsweise mit S. argillaceus DSM 14030, Umsatzraten bis zu 100 % der Verbindung (2) zu der Verbindung (1) erreicht.

### Beispiel 4

### Biotransformation mit Bacillus simplex DSM 14028, B. megaterium DSM 333 und B. megaterium DSM 339

Beispielhaftes Protokoll der Biotransformation für die Herstellung der Verbindung (1) aus der Verbindung (2).

### Beispiel 4a)

### Herstellung der primären Vorkulturen von Bacillus simplex DSM 14028, B. megaterium DSM 333 und B. megaterium DSM 339

Zur Herstellung der primären Vorkulturen wurden Bacillus simplex DSM 14028, B. megaterium DSM 333 und B. megaterium DSM 339 in folgendem Medium angezogen: TSB Medium (Medium 2): Trypticase Soy Broth (Difco) (30 g/l), ad 1 Liter Leitungswasser. Das Medium wurde bei 121°C und 1,1 bar Überdruck für 20 Minuten sterilisiert.

2 ml einer Bakteriensuspension in 50 % Glycerin wurden als Inokulum für 150 ml Medium 2 in einem 1000 ml Erlenmeyerkolben angesetzt und 48 Stunden bei 240 U/min auf einer Rundschüttelmaschine inkubiert. Dieses Medium wurde entweder benutzt, um neue Glycerinkonserven herzustellen, die bei 20°C aufbewahrt wurden, oder es diente als Inokulum für die Produktionskulturen (siehe 4b).

### Beispiel 4b)

### Produktionskultur (10 Liter Maßstab)

Zur Herstellung der Produktionskultur wurde 1 x 150 ml einer wie unter 4a) beschriebenen Vorkultur als Inokulum für 10 Liter Medium 3 (LB Broth Medium, Firma Sigma) eingesetzt. Vor dem Beimpfen wurde dem Medium 1 ml Entschäumer SAG 5693 (Union Carbide, USA) pro Liter zugesetzt, und für 30 Minuten bei 1,1 bar mit Dampf sterilisiert. Diese Produktionskultur wurde 90 Stunden bei 28°C bei einer Rührgeschwindigkeit von 300 U/min und mit einer Belüftungsrate von 0,3 vvm in einem 10 1 Giovanola Rührfermenter (Blattrührer) inkubiert. Die Verbindung (2) wurde in einer Endkonzentration von 50 - 250 mg/l Medium in methanolischer Lösung (2,5 g in 60 ml Methanol gelöst) zu Beginn der Fermentation zugesetzt. Zur Verfolgung des Biotransformations-Prozesses dienten tägliche Proben von 50 ml, die steril entnommen und mit Hilfe der analytischen HPLC untersucht wurden. Unter diesen Bedingungen wurden, vorzugsweise mit B. simplex DSM14028, Umsatzraten bis zu 60 % der Verbindung (2) zu der Verbindung (1) erreicht.

### Beispiel 5

### Analytische HPLC

Zur Detektion während der Biotransformation wurden analytische HPLC-Methoden mit UV/visueller (HPLC-UV/Vis) und mit massenspektrometrischer Detektion eingesetzt.

Vor der HPLC-Analyse wurden die Proben in MeOH gelöst und steril filtriert.

HPLC-MS wurden an einem HP1100 HPLC-System, gekoppelt an einem Micromass-LCT Massenspektrometer (Micromass, Manchester, Großbritannien) im ESI+ Modus durchgeführt. Massenspektren wurden im Bereich zwischen 200 und 1200 aufgezeichnet. Die Verbindung (1) hatte eine Retentionszeit von 4.08 Minuten. Das verwendete HP1100-System operierte mit folgenden Parametern: Stationäre Phase: Waters Symmetry C18, 3.5 um 2.1x50mm Säule, Mobile Phase: Gradient Wasser(A), Acetonitril (B) + 0.1 % Ameisensäure (0-1 Minuten 100 % A ; 1-5 Minuten linearer Gradient auf 10 % A /90 % B ; 5-6 Minuten 10 % A / 90 % B, 6-6.10 Minuten 90 % B - 100 % B). Die Verbindung (1) eluierte bei 4.08 Minuten. Der Nachweis erfolgte anhand der protonierten Molekülionen (M + H)⁺.

HPLC-UV/Vis-Analysen wurden mit Hilfe eines Hewlett Packard Series 1100 analytical HPLC system (HP, Waldbronn, Deutschland), bestehend aus einem G 1312A binären Pumpensystem, einem G 1315A Diodenarray-Detektor, einer G 1316A Säulentemperiersystem, einem G 1322A Entgasersystem und einem G 1313A Autoinjektor durchgeführt. Als mobile Phase wurde 0.01 % H₃PO₄: Acetonitril (ACN) bei einem Fluss von 1 ml/Minuten verwendet, während eine Merck (Darmstadt, Deutschland) Lichrospher RP 18 Säule (125 x 4 mm, Partikelgröße 5 µm) als stationäre Phase diente. Die Proben wurden an einem kontinuierlichen linearen Gradienten (0 % ACN bis 100 % ACN in 10 Minuten), gefolgt von isokratischer Elution (5 Minuten bei 100 % ACN) aufgetrennt. HPLC-UV Chromatogramme wurden bei 210 nm (Detektion von Verunreinigungen) und 254 nm (optimale Detektion im UV-Maximum der Spinosyn-Derivate) mit einer jeweiligen Referenzwellenlänge von 550 nm bei einer Bandbreite von 80 nm aufgezeichnet. Die Diodenarray-Detektion im Bereich von 210-600 nm lieferte die HPLC/UV/Vis Spektren. Die Speicherung der Daten erfolgte mit Hilfe der HP ChemStation Software. Die Verbindung (2) hatte hier eine Retentionszeit von 7.85-7-9 Minuten, während die Verbindung (1) bei einer Retentionszeit von 8.21 - 8.27 Minuten eluierte.

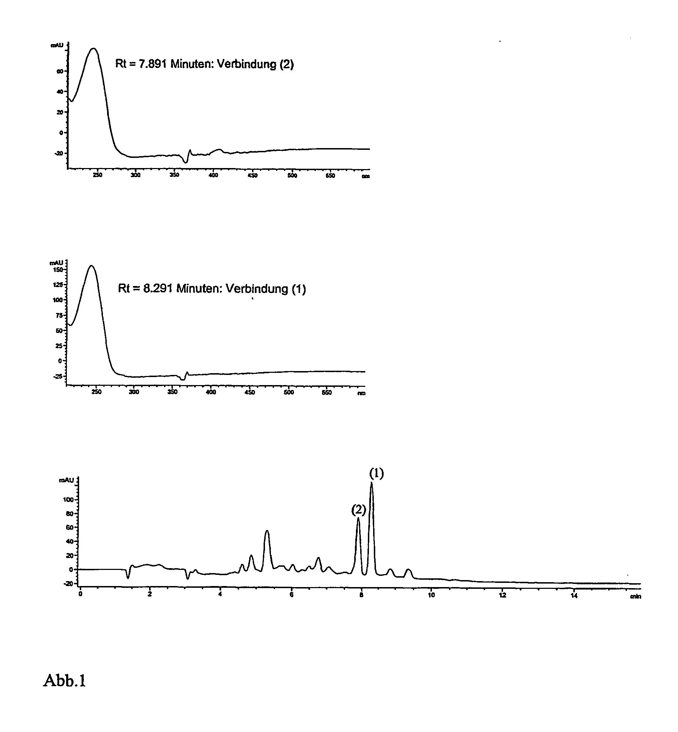

Für den analytischen Nachweis der Verbindung (2) und der Verbindung (1) in Fermentationsproben wurden interne und externe Standards der Reinsubstanzen verwendet, und die Detektion erfolgte bei übereinstimmenden Retentionszeiten in den beiden unterschiedlichen analytischen Systemen, einschließlich der jeweiligen HPLC-UV/Vis- und HPLC-MS- Spektren. Die Abbildung 1 zeigt ein HPLC-UV Chromatogramm des Rohextraktes bei 254 nm und HPLC-/UV-Spektren (Diodenarray; Rt = Retentionszeiten) der Verbindung (2) und der Verbindung (1) aus einer Fermentationsprobe von S. argillaceus (GS-Medium) nach 146 Stunden Fermentationsdauer.

### Beispiel 6a)

### Extraktion und präparative Reindarstellung der Verbindung (1) aus Schüttelkulturen

Zehn mit 50 mg/l Verbindung (2) versetzte 150 ml-Kulturen des Stammes (S. argillaceus DSM 14030) in 1000 ml Erlenmeyerkolben wurden nach 240 Stunden geerntet, und die Kulturbrühen wurden vereinigt und gefriergetrocknet. Die nach Gefriertrocknung erhaltenen Rückstände wurden zweimal für jeweils 30 Minuten im Ultraschallbad extrahiert. Die Überstände wurden abfiltriert, vereinigt und im Vakuum am Rotationsverdampfer bis zur Trockne eingeengt. Die Rückstände wurden in 100 ml Wasser:Ethylacetat (EtOAc) 1:1 zurückgelöst. Die organischen Ethylacetatphasen wurde abgetrennt, über wasserfreiem Natriumsulfat getrocknet und im Rotationsverdampfer bis zur Trockne eingeengt. Die wässrige Phase wurde noch zweimal mit jeweils 50 ml Ethylacetat versetzt, die organischen Phasen abgetrennt und mit der ersten Phase vereinigt. Aus den vereinigten organischen Phasen der Extraktion wurden ca. 300 mg eines öligen Rohproduktes erhalten, welche in 2 ml MeOH gelöst und über eine Baker (Deventer, Niederlande) Bond Elut C18 1 ml Festphasenextraktionskartusche filtriert wurde. Das Filtrat wurde unmittelbar für die präparative HPLC eingesetzt.

Die präparative HPLC wurde bei 22°C betrieben. Das verwendete System der Fa. Gilson Abimed (Ratingen, Deutschland) bestand aus Gilson Unipoint Software, einem 306 binären Pumpensystem, einem 205 Fraktionssammler, einem 119 UV-Vis Detektor, einem 806 manometrischen Modul und einem 811C dynamischen Mixer. Eine Merck (Darmstadt, Deutschland) LichroSorb RP18 Säule (Partikelgröße 7 µm; Säulendimensionen 250 x 25 mm) wurde als stationäre Phase verwendet Als mobile Phase diente ein Gradient von 0.1 % wässriger Trifluoressigsäure gegen Acetonitril (ACN) mit einem kontinuierlichen Fluss von 10 ml/Minuten. unter folgendem Elutionsprofil: Linearer Gradient von 20 % bis 50 % ACN in 35 Minuten; danach isokratische Bedingungen bei 50 % ACN für 25 Minuten, gefolgt von einem linearen Gradienten von 50 % ACN zu 100 % ACN in 40 Minuten. Aliquots von 10 ml wurden fraktioniert und je nach UV-Adsorption bei 210 nm vereinigt. Retentionszeiten (Rt) waren 77-82 Minuten für die Verbindung (2) und 91-97 Minuten für die Verbindung (1).

### Beispiel 6b)

### Extraktion und Isolierung der Verbindung (1) aus Rührfermenter-Kulturen (10 Liter Maßstab)

Die Myzelien aus Fermentationen des Stammes B. simplex DSM 14028 im 10 Liter Maßstab wurden durch Zentrifugation (10 Minuten bei 1000 x g) vom Überstand abgetrennt und direkt mit 2 x 200 ml Methanol extrahiert. Die Methanolextrakte wurden bis zur Trockne eingeengt und lieferten ein öliges Zwischenprodukt. Dieses Produkt wurde wie weiter unten beschrieben über Flash-Chromatografie an Kieselgel Material weiterbearbeitet.

Der nach Zentrifugation erhaltene Kulturüberstand wurde über eine 6 x 18 cm Lewapol (500ml) OC 1064 (Bayer AG, Leverkusen, Deutschland) Adsorberharzsäule gegeben. Diese Säule wurde mit 11 Wasser gewaschen und aufeinanderfolgend mit 21 70% Methanol und 1 1 Aceton eluiert. Die organischen Eluate wurden bis zu einem wässrigen Rückstand am Rotationsverdampfer eingeengt. Die Masse des Produktes fand sich in der Aceton-Fraktion wieder, die anschließend über Flash Chromatographie - wie weiter unten beschrieben - weiter bearbeitet wurde.

Die öligen Zwischenprodukte aus den Extraktionen von Myzel und Überstand wurden dazu in möglichst wenig tert-Butylmethylether (TMBE) gelöst und an 400 ml Kieselgel 60 (0.040 - 0.063 mm, Merck Darmstadt, Deutschland) gebunden. Der TMBE wurde vorsichtig am Rotationsverdampfer abgedampft. Anschließend wurden die an das Trägermaterial gebundenen Zwischenprodukte trocken auf 100 ml Kieselgel in ein 500 ml SIM-Modul (Biotage) gepackt. Es wurde nacheinander isokratisch mit 2 1 Cyclohexan (CH), gefolgt von 1,6 1 eines linearen Gradienten von Cyclohexan gegen TBME eluiert.

Die mit CH:TBME eluierten Fraktionen enthielten das stark angereicherte Produkt neben Restmengen an nicht umgesetztem Aglykon und anderen Inhaltsstoffen. Diese Fraktionen wurden am Rotationsverdampfer eingeengt und zur Isolierung der Verbindung (1) auf die präparative HPLC gegeben.

Die Isolierung der Verbindung (1) erfolgte unter Verwendung des in Beispiel 6a beschriebenen präparativen HPLC-Systems unter Verwendung einer MZ Analysentechnik Kromasil 100 C8 (7 µm, 250 x 40 mm)-Säule als stationärer Phase und 0.1 % Trifluoressigsäure: Acetonitril (ACN) als mobiler Phase bei einem kontinuierlichen Fluss von 10 ml/Minuten in folgendem Gradienten: 30 % ACN für 20 Minuten, gefolgt von linearem Gradienten (30 % - 50 % ACN in 60 Minuten), dann wiederum linearer Gradient von 50 % ACN zu 100 % ACN in 42 Minuten.

Aliquots von 10 ml wurden fraktioniert und nachträglich je nach UV-Absorption bei 210 nm vereinigt. Retentionszeiten (Rt) waren 105-111 Minuten für die Verbindung (2) und 115-119 Minuten für die Verbindung (1).

Die Aufarbeitung und Isolierung aus Fermentationen im größeren Maßstab erfolgte analog zu diesem Verfahren, wobei Extraktions-, Elutions- und Säulenbettvolumina entsprechend erhöht (d.h. den Volumina der Produktionskulturen angepasst) und die Zwischenprodukte ggf. vor der abschließenden HPLC-Trennung aliquotiert wurden.

### Beispiel 7

### Strukturaufklärung der Verbindung (1)

Die Strukturaufklärung der Verbindung (1) wurde mit Hilfe der 1D und 2D-Kernresonanzspektroskopie (NMR) und der positiven Elektrospray-Massenspektrometrie (+ESI-MS) durchgeführt. NMR-Spektren wurden an einem Bruker DMX500 Spektrometer bei 302 K in DMSO gemessen. MS Spektren wurden an einem LCT ESI-TOF Gerät der Fa. Micromass gemessen*.*

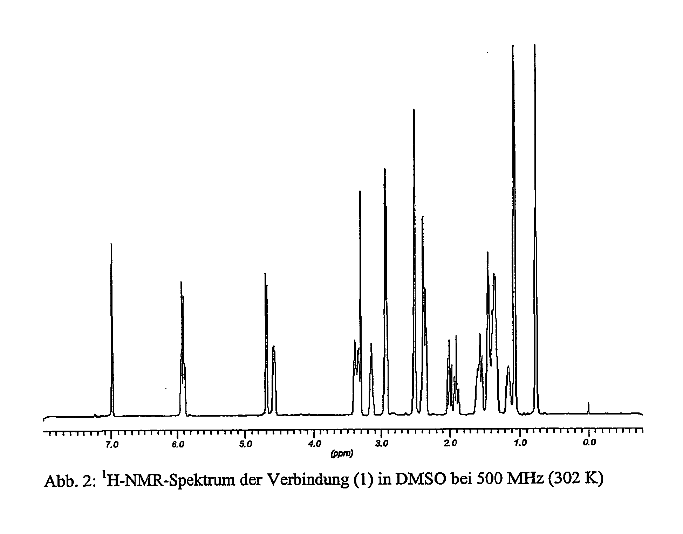

Die durch (ES+I)-MS bestimmte Masse betrug 400 (C₂₄H₃₂O₅) (entsprechend einer gemessenen Molmasse von 401 (m/z+H)). Die durch MS-Hochauflösung (ESI+) bestimmte Masse betrug 401.2340 (berechnet: 401.2328). Die NMR-Daten sind in der folgenden Tabelle, zusammengefasst, und das Protonenspektrum ist in Abb. 2 abgebildet.

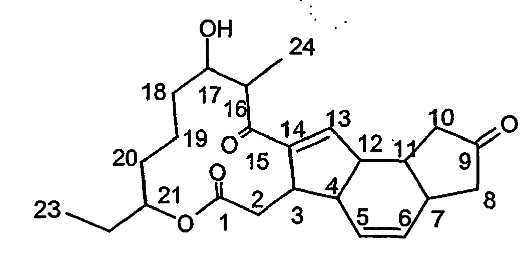

| **Atom-Nr.** | **C (ppm)** | **H (ppm)** |
|---|---|---|
| 1 | 172,2 | - |
| 2 | 33,6 | 2,51; 2,93 |
| 3 | 48,7 | 2,94 |
| 4 | 40,6 | 3,39 |
| 5 | 129,1 | 5,91 |
| 6 | 129,0 | 5,94 |
| 7 | 39,9 | 2,38 |
| 8 | 43,1 | 1,91; 2,38 |
| 9 | 219,0 | - |
| 10 | 42,7 | 2,00; 2,34 |
| 11 | 44,0 | 1 ,35 |
| 12 | 47,9 | 2,93 |
| 13 | 147,1 | 7,00 |
| 14 | 144,2 | - |
| 15 | 203,6 | - |
| 16 | 47,8 | 3,14 |
| 17 | 70,9 | 3,35 |
| 18 | 34,9 | 1,37; 1,34 |
| 19 | 21,7 | 1,15; 1,59 |
| 20 | 29,5 | 1 ,54; 1,34 |
| 21 | 76,3 | 4,60 |
| 22 | 28,1 | 1,44 |
| 23 | 9,2 | 0,76 |
| 24 | 15,9 | 1,07 |
| 25 | | 3,74 (OH) |

| | | |
|---|---|---|
| ¹H und ¹³C chemische Verschiebungen der Verbindung (1) in DMSO-d₆ gemessen bei 302 K | | |

Die Bestimmung der optischen Rotation erfolget an einem Perkin-Elmer Polarimeter Model 341 bei 20°C und 589nm (10cm Küvettenlänge, Lösemittel Methanol). Der optischer Drehwinkel der Verbindung (1): (NaD, 589 nm) betrug in MeOH: -272.2°.

### Beispiel 9

### Charakterisierung der verwendeten Stämme

### a) Streptomyces argillaceus DSM 14030:

Dieser Stamm wurde als Mithramycinproduzent und als Typ-Stamm der neuen Streptomyces Art S. argillaceus bei der American Type Culture Collection unter der Zugangsnummer ATCC 12956 von der Pfizer Inc., New York, USA hinterlegt (S. argillaceus ATCC 12956). Die Stammbeschreibung kann der US-A 3 646 194 entnommen werden. Die Kultur wurde nochmals am 08.02.2001 unter der Hinterlegungsnummer DSM 14030 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.

### b) Streptomyces scabies (BS 2134):

Der Stamm BS 2134 wurde aus einer in Rheinland-Pfalz (Deutschland) gesammelten Bodenprobe isoliert. Die Kultur wurde am 08.02.2001 unter der Hinterlegungsnummer DSM 14029 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.

Der Stamm BS 2134 wurde als Streptomyces scabies klassifiziert. Die Ergebnisse basieren auf Vergleich mit Streptomyces scabies DSM 40478 unter Verwendung der Methoden des Internationalen Streptomyces-Projekts (ISP) zur Charakterisierung und Identifizierung von Streptomyces-Spezies (Küster E. (1972), Int. J. Syst. Bacteriology, 22: 139), sowie mit Hilfe von Sequenzanalysen der 16S rDNA (Maidak et. al. (1996), Nucleic Acids Res., 24: 82). Die Ergebnisse sind in der folgenden Tabelle zusammengefasst. Nach der 16S rDNA-Sequenzanalyse zeigt der Stamm zu 100 % Übereinstimmung zu S. annulatus, der dem gleichen Speziescluster wie S. scabies zugeordnet wird. Da die morphologischen und physiologischen Merkmale gemäß der ISP-Beschreibung jedoch besser mit S. scabies übereinstimmen, wurde der Stamm als S. scabies identifiziert.

ISP-Beschreibung. Sporenkettenmorphologie: Rectiflexibiles, kurze Sporenketten mit maximal 10 Sporen. Das Luftmyzel gehört zur Grauen Reihe, das Substratmyzel ist gelb bis grau-grün. Melaminpigmente werden nicht gebildet. Saccharose, Inositol und Raffinose werden nicht verwertet.

### c) Streptomyces spec. (BA 312):

Der Stamm BA 312 wurde aus einer in Rheinland-Pfalz (Deutschland) gesammelten Bodenprobe isoliert. Die Kultur wurde am 27.02.2001 unter der Hinterlegungsnummer DSM 14077 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.

Der Stamm BA 312 konnte weder molekularbiologisch noch chemotaxonomisch einer bekannten Streptomyces-Spezies zugeordnet werden. Die physiologischen Testergebnisse (Zuckerverwertung, siehe folgende Tabelle) zeigen Ähnlichkeit zu S. ochraceiscleroticus, die morphologischen Merkmale (graues Luftmyzel, gerade Sporenketten, gelbe Substratmyzelfarbe, keine Melanisierung) und die 16S rDNA-Analyse stimmen jedoch nicht mit S. ochraceiscleroticus überein, so dass der Stamm als S. spec. klassifiziert werden muss (siehe folgende Tabelle).

### d) Streptomyces mirabilis (BA 579):

Der Stamm BA 579 wurde aus einer in Rheinland-Pfalz (Deutschland) gesammelten Bodenprobe isoliert. Die Kultur wurde am 27.02.2001 unter der Hinterlegungsnummer DSM 14078 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.

Aufgrund der hohen 16S rDNA-Sequenzähnlichkeit und der morphologischen Merkmale wurde dieser Stamm als S. mirabilis klassifiziert. Der Stamm unterscheidet sich aber in der ISP-Zuckerverwertung durch das Wachstum mit Saccharose und Raffinose sowie das Fehlen der Melaninbildung (siehe folgende Tabelle).

ISP-Beschreibung: Graues Luftmyzel, Sporenkettenmorphologie: Section Spirales, Sporulation ist nur schwach ausgeprägt. Die Substratmyzelfarbe ist grau-gelb und wird olivebraun bis braun. In Pepton-Hefe-Eisen Agar werden Melaminpigmente gebildet. Saccharose und Raffinose werden nicht verwertet.

### e) Streptomyces pseudovenecuelae (WS 2199):

Der Stamm WS 2199 wurde aus einer wässrigen Probe (Deutschland) isoliert. Die Kultur wurde am 27.02.2001 unter der Hinterlegungsnummer DSM 14079 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.

Aufgrund der hohen 16S r DNA-Sequenzähnlichkeit, der morphologischen Merkmale und der physiologischen Testergebnisse konnte dieser Stamm als S. pseudovenecuelae klassifiziert werden. Eine Ausnahme bildet die Luftmyzelfarbe, die nicht rot-grau, sondern nur grau ist (siehe folgende Tabelle).

ISP-Beschreibung: Luftmyzelfarbe: Rote Reihe, Grau ist auch schon berichtet worden, Sporenkettenmorphologie: Section Rectiflexibiles. Sporenketten sehr lang (bis zu 50 Sporen). Melanisierung. D-Glucose, L-Arabinose, Saccharose, D-Xylose, Inositiol, D-Mannitol, Fructose, Rhamnose und Raffinose werden verwertet.

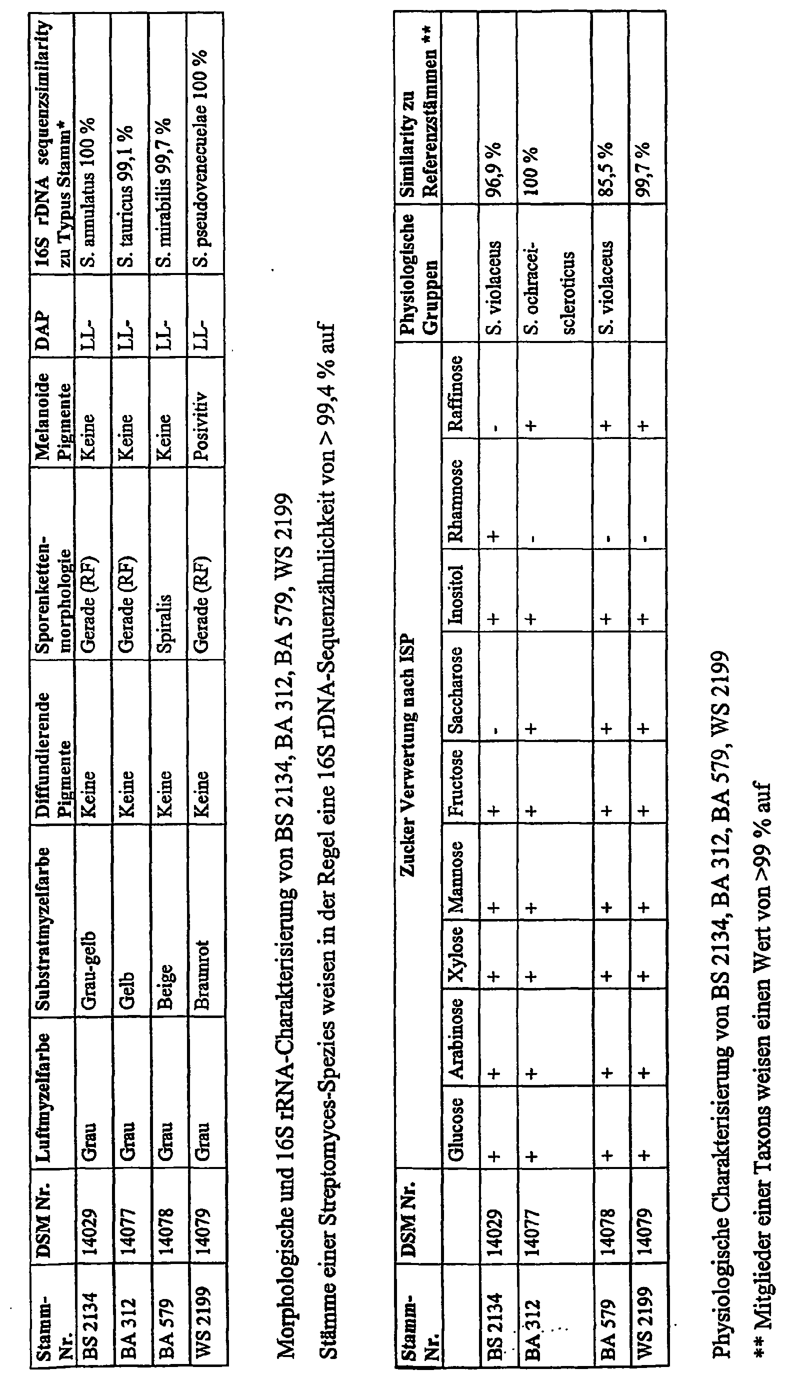

### f) Bacillus simplex DSM 14028:

Bacillus simplex DSM 14028 wurde als Bacillus megaterium NRS 610 in die Stammsammlung Collection of Nathan R Smith, U.S. Department of Agriculture, Washington, D.C., USA aufgenommen und von Priest et al. als Bacillus simplex neu beschrieben. Er wurde von der Deutschen Sammlung für Mikroorganismen als DSM 1318 in die Sammlung aufgenommen (Priest, F. G., Goodfellow, M., Todd, C., A numerical classification of the genus Bacillus. J. Gen. Microbiol. 134: 1847-1882, 1988). Der Stamm wurde nochmals am 08.02.2001 unter der Hinterlegungsnummer DSM 14028 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.

### g) Bacillus megaterium DSM 333:

Bacillus megaterium DSM 333 wurde von D. Claus unter der Hinterlegungsnummer DSM 333 bei der Deutschen Sammlung für Mikroorganismen hinterlegt und von Hunger et al. beschrieben (Hunger, W., Claus, D.: Taxonomic studies on Bacillus megaterium and on agarolytic Bacillus strains, S. 217-239, In Berkeley, R.C.W., Goodfellow, M (eds.) The aerobic endospore-forming bacteria: classification and identification. Academic Press, London 1981).

### h) Bacillus megaterium DSM 339:

Bacillus megaterium DSM 339 wurde vom Institut für Mikrobiologie der Universität Göttingen unter der Hinterlegungsnummer DSM 339 bei der Deutschen Sammlung für Mikroorganismen hinterlegt. Die Taxonomie des Stammes ist von Hunger et al. beschrieben (Hunger, W., Claus, D.: Taxonomic studies on Bacillus megaterium and on agarolytic Bacillus strains, S. 217-239, In Berkeley, R.C.W., Goodfellow, M (eds.). The aerobic endospore-forming bacteria: classification and identification. Academic Press, London 1981).

### i) Zygorhynchus moelleri Vuill. - Stamm WP 0796:

Der Stamm wurde von Dr. H.-G. Wetzstein (Bayer AG) 1994 aus einer in Deutschland gesammelten Bodenprobe isoliert und von Dr. P. Hofmann (DSMZ) morphologisch charakterisiert. Über eine mikroskopische Kontrolle der bei der DSMZ hinterlegten Kultur wurde diese Taxonomie unter Zuhilfenahme des einschlägigen Bestimmungs-Schlüssels in K.H. Domsch et al. (1980), Compendium of soil fungi Vol. 2. ICW Verlag (unter Zygorrhynchus moelleri) bestätigt. Der aktuell gültige Gattungsname wird allerdings nach Hawksworth, D.L. et al. (eds.): Ainsworth & Bisby's Dictionary of the Fungi, CAB International, London, 8^{th} edition (1996) als Zygorhynchus (statt Zygorrhynchus) wiedergegegeben. Die Kultur wurde am 21.03.2001 unter der Hinterlegungsnummer DSM 14198 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.

### j) Mucor circinelloides Van Tiegh - Stamm WP 0799:

Der Stamm wurde von Dr. H.-G. Wetzstein (Bayer AG) 1994 aus einer in Deutschland gesammelten Bodenprobe isoliert und von Dr. P. Hofmann (DSMZ) morphologisch charakterisiert. Über eine mikroskopische Kontrolle der bei der DSMZ hinterlegten Kultur wurde diese Taxonomie unter Zuhilfenahme des einschlägigen Bestimmungs-Schlüssels in K.H. Domsch et al. (1980) Compendium of soil fungi Vol. 2. ICW Verlag (unter Mucor circillenoides) bestätigt. Die Kultur wurde am 21.03.2001 unter der Hinterlegungsnummer DSM 14199 bei der Deutschen Sammlung von Mikroorganismen und Zellkulturen GmbH (DSMZ), Mascheroder Weg 1b, D-38124 Braunschweig, Deutschland, in Übereinstimmung mit den Bestimmungen des Budapester Vertrages hinterlegt.

### Beispiel 10

### Chemische Darstellung der Verbindung (1) durch Pyridiniumdichromat-Oxidation

Die Verbindung (1) wurde durch Pyridiniumdichromat-Oxidation aus der Verbindung (2) hergestellt: 46.55 g (115.6 mmol) der Verbindung (2) wurden unter Inertgas in 1100 ml abs. Dichlormethan gelöst und mit 43.51 g (115.6 mmol) Pyridiniumdichromat versetzt. Nach 4 Stunden Rühren bei 25°C und Zugabe von 900 ml Diethylether wurden die ausgefallenen Chromsalze abfiltriert und das Filtrat im Vakuum eingeengt. Säulenchromatographie an Kieselgel (Eluent: Cyclohexan/Essigsäureethylester 1:1, dann 100 % Essigsäureethylester) lieferte 3.68 g 9,17-Diketospinosyn-Aglykon und 23.40 g einer ca. 9:1-Mischung der Verbindung (2) und 17-Ketospinosyn-Aglykon neben 11.74 g zurückgewonnener Verbindung (2). Durch Umkristallisation der Mischung in Cyclohexan/Essigsäureethylester wird die Verbindung (1) auf> 98 % angereichert. Man erhält 20.78 g der Verbindung (1) als farblose Kristalle. - Verbindung (1): DC: *R*_{f} (SiO₂, Essigsäureethylester) = 0.44 - ¹H-NMR: CDCl₃, δ = 6.77 (s, 13-H); 5.97 (d, 6-H); 5.88 (m, 5-H); 4.72 (m, 21-H); 3.69 (m, 17-H) u. a. - LC/ESI-MS: *m*/*z* = 401 (25 %) [M]⁺, 289 (100 %). Die auf diese Weise dargestellte Verbindung (1) ist in allen spektroskopischen Daten identisch mit der durch Biokonversion dargestellten Verbindung (1). - 17-Ketospinosyn-Aglykon: DC: *R*_{f} (SiO₂, Essigsäureethylester) = 0.40 - ¹H-NMR: CDCl₃, δ = 6.97 (s, 13-H); 5.90 (d, 6-H); 5.79 (m, 5-H); 4.85 (m, 21-H); 4.45 (m, 9-H); 4.25 (q, 16-H) u. a. - LC/ESI-MS: *m*/*z* = 401 (100 %) [M+H]⁺, 273 (70 %). - 9,17-Diketospinosyn-Aglykon: DC: *R*_{f} (SiO₂, Essigsäureethylester) = 0.64. - ¹H-NMR: CDCl₃, δ = 6.92 (s, 13-H); 5.97 (d, 6-H); 5.87 (m, 5-H); 4.85 (m, 21-H); 4.25 (q, 16-H) u. a. - LC/ESI-MS: *m*/*z* = 399 (100 %) [M+H]⁺.

### Beispiel 10a)

### Chemische Darstellung der Verbindung (1) durch Swern-Oxidation

Die Verbindung (1) wurde alternativ durch Swern-Oxidation (Variante nach Corey und Khim (1972), J. Am. Chem. Soc., 94: 7586) aus der Verbindung (2) hergestellt: 133 mg (1 mmol) N-Chlorsuccinimid wurden unter Inertgas in 10 ml abs. Dichlormethan suspendiert und bei - 70°C mit 66 mg (1.07 mmol) Dimethylsulfid versetzt. Nach 30 min. Rühren wurde eine Lösung von 402 mg (1 mmol) der Verbindung (1) in 2 ml abs. Dichlormethan langsam zugetropft. Nach 2 Stunden Rühren bei - 70°C wurden 132 µl (0.95 mmol) Triethylamin zugetropft und die Reaktionsmischung innerhalb von 16 Stunden auf Raumtemperatur aufgewärmt. Nach Verdünnen mit 100 ml Dichlormethan wurde nacheinander mit 100 ml Wasser und 100 ml gesättigter wässriger Natriumchlorid-Lösung gewaschen, über Natriumsulfat getrocknet und im Vakuum eingeengt. Säulenchromatographie an Kieselgel (Eluent: Cyclohexan/Essigsäureethylester 2:1) lieferte 246 mg Verbindung (1) neben 37 mg 9,17-Diketospinosyn-Aglykon und 120 mg zurückgewonnener Verbindung (2). Die auf diese Weise dargestellte Verbindung (1) ist in allen spektroskopischen Daten identisch mit der durch Pyridiniumdichromat-Oxidation bzw. Biokonversion dargestellten Verbindung (1).

### Beispiel 10b)

### Chemische Darstellung von 5,6-Dihydro-9-Keto-Spinosyn-Aglykon (Verbindung (3))

Die Verbindung (3) wurde analog zu Beispiel 10a) durch Pyridiniumdichromat-Oxidation aus der Verbindung (4) hergestellt: 202 mg (0.5 mmol) Verbindung (4) wurden unter Inertgas in 5 ml abs. Dichlormethan gelöst und mit 188 mg (0.5 mmol) Pyridiniumdichromat versetzt. Nach 4 Stunden Rühren bei 25°C und Zugabe von 5 ml Diethylether wurden die ausgefallenen Chromsalze abfiltriert und das Filtrat im Vakuum eingeengt. Säulenchromatographie an Kieselgel (Eluent: Cyclohexan/Essigsäureethylester 1:1, dann 100 % Essigsäureethylester) lieferte 24 mg 5,6-Dihydro-9,17-Diketo-Spinosyn-Aglykon und 103 mg Verbindung (3) neben 41 mg zurückgewonnenem Edukt Verbindung (4). - Verbindung (3): DC: *R*_{f} (SiO₂, Essigsäureethylester) = 0.44 - ¹H-NMR: CDCl₃, δ = 6.83 (s, 13-H); 4.68 (m, 21-H); 3.69 (m, 17-H) u. a. - LC/ESI-MS: *m*/*z* = 403 (23 %) [M+H]⁺, 291 (100 %). - 5,6-Dihydro-9,17-Diketospinosyn-Aglykon: DC: *R*_{f} (SiO₂, Essigsäureethylester) =0.64. - ¹H-NMR: CDCl₃, δ = 6.99 (s, 13-H); 4.82 (m, 21-H); 4.23 (q, 16-H) u. a. - LC/ESI-MS: *m*/*z* = 423 (100 %) [M+Na]⁺.

### Beispiel 11

### Darstellung des 9-Keto-Spinosyn A-9-Pseudoaglykons

### a) Synthese des Trichloracetimidats (vgl. auch Methode bei G. R. Duffin et al. (2000), J. Chem. Soc., Perkin Trans. 1: 2237-2242):

200 mg (1.256 mmol) α-D-Forosamin wurden in 10 ml Methylenchlorid verrührt, mit 419.0 mg (2.902 mmol) Trichloracetonitril und 108,5 mg (0.333 mmol) Cäsium-carbonat versetzt und ca. zwei Stunden bei Raumtemperatur gerührt. Anschließend wurde das Reaktionsgemisch mit Methylenchlorid verdünnt, mit wässriger Natriumhydrogencarbonat-Lösung gewaschen, die organische Phase über Magnesiumsulfat getrocknet und das Lösungsmittel im Vakuum abgezogen. Man erhält 303 mg (79,5 %) Trichloracetimidat, welches unmittelbar für die Glykosidierungsreaktion (b) verwendet werden kann.
C₁₀H₁₇Cl₃N₂O₂ (303.6)
LC/MS: *m*/*z* = 303 (100 %) [M]⁺

### b) Glykosidierung von Verbindung (1) mit Trichloracetimidat (vgl. auch Methode bei G. R. Duffin et al. (2000), J. Chem. Soc., Perkin Trans. 1: 2237-2242):

100 mg (0.250 mmol) Verbindung (1) wurden in 2 ml Methylenchlorid verrührt, mit 50 mg Molsieb 4A und 151 mg (0.500 mmol) frischem Trichloracetimidat (a) versetzt. Anschließend wurden 49.4 mg (0.348 mmol) Bortrifluorid-Etherat hinzugegeben, und das Reaktionsgemisch wurde ca. 18 Stunden bei Raumtemperatur gerührt. Nach Abtrennen des Molsiebs 4A wurde der Rückstand mit wenig Methylenchlorid verdünnt und mit wässriger Natriumhydrogencarbonat-Lösung gewaschen. Die organische Phase wurde über Magnesiumsulfat getrocknet und das Lösungsmittel im Vakuum abgezogen.
C₃₂H₄₇NO₆ (541.7)
LC/MS: *m*/*z =* 542 (100 %) [M]⁺ (vgl. WO 97/00265 und US-A 6 001 981)

## Patentansprüche

1. Verbindungen der Formel (I) 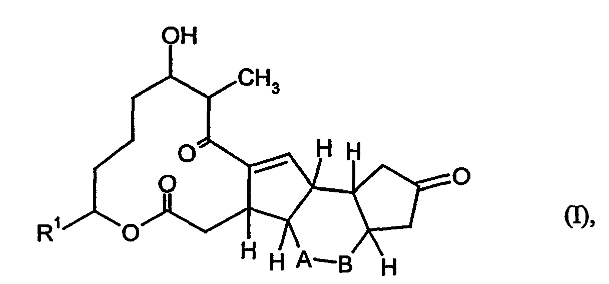 in welcher
R¹ für Methyl oder Ethyl steht, und
A-B für eine der folgenden Gruppen steht: -HC=CH-, -HC=C(CH₃)-, -H₂C-CH₂-, -H₂C-CH(CH₃)-.

2. Verbindungen gemäß Anspruch 1, **dadurch gekennzeichnet, dass**
R¹ für Ethyl steht.

3. Verbindungen gemäß Anspruch 1 oder 2, **dadurch gekennzeichnet, dass**
A-B für die Gruppe -HC=CH- steht.

4. Verfahren zum Herstellen von Verbindungen gemäß einem der Ansprüche 1 bis 3, **dadurch gekennzeichnet, dass** man Verbindungen der allgemeinen Formel (II) 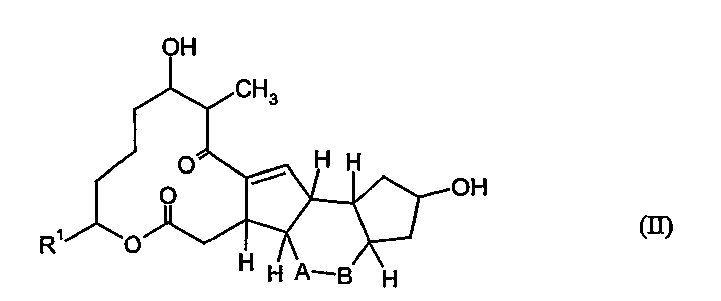 worin
R¹ und A-B die in einem der Ansprüche 1 bis 3 angegebenen Bedeutungen haben,
mit einem Oxidationsmittel, gegebenenfalls in Gegenwart eines Verdünnungsmittels, umsetzt.

5. Verfahren gemäß Anspruch 4, **dadurch gekennzeichnet, dass** man als Oxidationsmittel Pyridiniumdichromat oder N-Halogensuccinimide in Gegenwart von Dimethylsulfid einsetzt.

6. Verfahren gemäß Anspruch 4 oder 5, **dadurch gekennzeichnet, dass** man als Verdünnungsmittel Dichlormethan einsetzt.

7. Verfahren zum Herstellen von Verbindungen gemäß einem der Ansprüche 1 bis 3, **dadurch gekennzeichnet, dass** man Verbindungen der allgemeinen Formel (II) 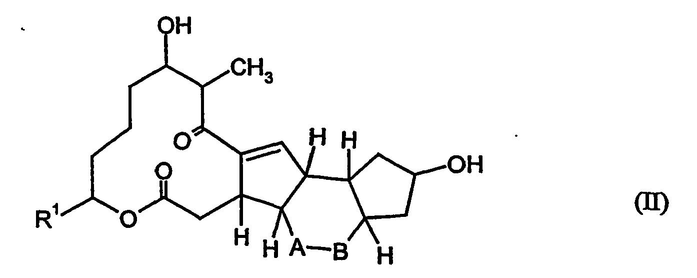 worin
R¹ und A-B die in einem der Ansprüche 1 bis 3 angegebenen Bedeutungen haben,
mit einem Mikroorganismus in einem wässrigen Nährmedium unter aeroben Bedingungen oder mit einem daraus hergestellten Enzymextrakt oder mit einem oder mehreren daraus isolierten Enzymen in Kontakt bringt.

8. Verfahren gemäß Anspruch 7, **dadurch gekennzeichnet, dass** man als Mikroorganismus einen Mikroorganismus aus der Gattung Bacillus, aus der Gruppe der Actinomyceten oder aus der Klasse der Zygomyceten einsetzt.

9. Verfahren gemäß Anspruch 8, **dadurch gekennzeichnet, dass** man einen Stamm aus der Art Bacillus megaterium oder Bacillus simplex einsetzt.

10. Verfahren gemäß Anspruch 9, **dadurch gekennzeichnet, dass** der Stamm die kennzeichnenden Merkmale der Stämme Bacillus megaterium DSM 339, Bacillus megaterium DSM 333 oder Bacillus simplex DSM 14028 aufweist.

11. Verfahren gemäß Anspruch 8, **dadurch gekennzeichnet, dass** man als Mikroorganismus aus der Gruppe der Actinomyceten einen Mikroorganismus aus der Gattung Streptomyces einsetzt.

12. Verfahren gemäß Anspruch 11, **dadurch gekennzeichnet, dass** man als Mikroorganismus aus der Gattung Streptomyces einen Stamm aus der Art Streptomyces argillaceus, Streptomyces scabies, Streptomyces mirabilis oder Streptomyces pseudovenecuelae einsetzt.

13. Verfahren gemäß Anspruch 12, **dadurch gekennzeichnet, dass** der Stamm die kennzeichnenden Merkmale der Stämme Streptomyces argillaceus DSM 14030, Streptomyces scabies DSM 14029, Streptomyces mirabilis DSM 14078 oder Streptomyces pseudovenecuelae DSM 14079 aufweist.

14. Verfahren gemäß Anspruch 8, **dadurch gekennzeichnet, dass** man als Mikroorganismus aus der Klasse der Zygomyceten einen Mikroorganismus aus der Gattung Zygorhynchus, insbesondere einen Stamm aus der Art Zygorhynchus moelleri einsetzt.

15. Verfahren gemäß Anspruch 14, **dadurch gekennzeichnet, dass** der Stamm die kennzeichnenden Merkmale des Stammes Zygorhynchus moelleri DSM 14198 aufweist.

16. Verfahren gemäß Anspruch 8, **dadurch gekennzeichnet, dass** man als Mikroorganismus aus der Klasse der Zygomyceten einen Mikroorganismus aus der Gattung Mucor, insbesondere einen Stamm aus der Art Mucor circinelloides einsetzt.

17. Verfahren gemäß Anspruch 16, **dadurch gekennzeichnet, dass** der Stamm die kennzeichnenden Merkmale des Stammes Mucor circinelloides DSM 14199 aufweist.

18. Verfahren gemäß einem der Ansprüche 4 bis 17, **dadurch gekennzeichnet, dass** man die Verbindungen gemäß einem der Ansprüche 1 bis 3 isoliert.

19. Verwendung von Verbindungen gemäß einem der Ansprüche 1 bis 3 zum Herstellen von Spinosyn-Derivaten.

## Claims

1. Compounds of the formula (I) 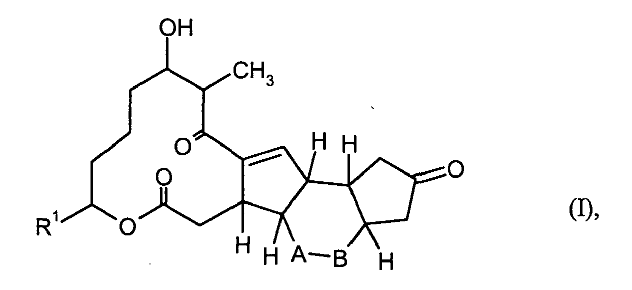 in which
R¹ represents methyl or ethyl, and
A-B represents one of the following groups: -HC=CH-, -HC=C(CH₃)-, -H₂C-CH₂-, -H₂C-CH(CH₃)-.

2. Compounds according to Claim 1, **characterized in that**
R¹ represents ethyl.

3. Compounds according to Claims 1 or 2, **characterized in that**
A-B represents the group -HC=CH-.

4. Process for the preparation of compounds according to any of Claims 1 to 3, **characterized in that** compounds of the general formula (II) 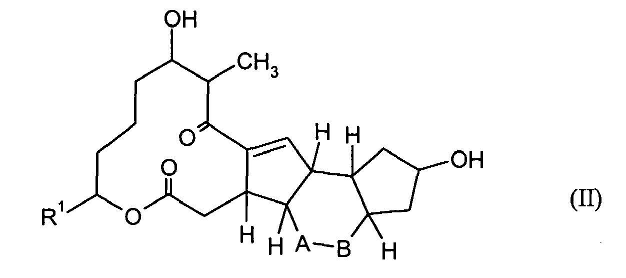 in which
R¹ and A-B have the meanings given in any of Claims 1 to 3,
are reacted with an oxidant, if appropriate in the presence of a diluent.

5. Process according to Claim 4, **characterized in that** the oxidant employed is pyridium dichromate or N-halosuccinimides in the presence of dimethyl sulfide.

6. Process according to Claim 4 or 5, **characterized in that** the diluent employed is dichloromethane.

7. Process for the production of compounds according to any of Claims 1 to 3, **characterized in that** compounds of the general formula (II) 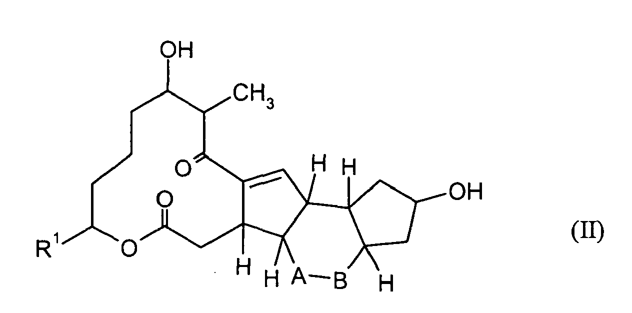 in which
R¹ and A-B have the meanings given in any of Claims 1 to 3,
are brought into contact with a microorganism in an aqueous nutrient medium under aerobic conditions or with an enzyme extract prepared therefrom or with one or more enzymes isolated therefrom.

8. Process according to Claim 7, **characterized in that** the microorganism employed is a microorganism from the genus Bacillus, from the group of the Actinomycetes or from the class of the Zygomycetes.

9. Process according to Claim 8, **characterized in that** a strain of the species Bacillus megaterium or Bacillus simplex is employed.

10. Process according to Claim 9, **characterized in that** the strain has the characterizing traits of the strains Bacillus megaterium DSM 339, Bacillus megaterium DSM 333 or Bacillus simplex DSM 14028.

11. Process according to Claim 8, **characterized in that** a microorganism from the genus Streptomyces is employed as microorganism from the group of the Actinomycetes.

12. Process according to Claim 11, **characterized in that** a strain from the species Streptomyces argillaceus, Streptomyces scabies, Streptomyces mirabilis or Streptomyces pseudovenecuelae is employed as microorganism from the genus Streptomyces.

13. Process according to Claim 12, **characterized in that** the strain has the characterizing traits of the strains Streptomyces argillaceus DSM 14030, Streptomyces scabies DSM 14029, Streptomyces mirabilis DSM 14078 or Streptomyces pseudovenecuelae DSM 14079.

14. Process according to Claim 8, **characterized in that** a microorganism from the genus Zygorhynchus, in particular a strain from the species Zygorhynchus moelleri, is employed as microorganism from the class of the Zygomycetes.

15. Process according to Claim 14, **characterized in that** the strain has the characterizing traits of the strain Zygorhynchus moelleri DSM 14198.

16. Process according to Claim 8, **characterized in that** a microorganism from the genus Mucor, in particular a strain from the species Mucor circinelloides, is employed as microorganism from the class of the Zygomycetes.

17. Process according to Claim 16, **characterized in that** the strain has the characterizing trait of the strain Mucor circinelloides DSM 14199.

18. Process according to any of Claims 4 to 17, **characterized in that** the compounds according to any of Claims 1 to 3 are isolated.

19. Use of compounds according to any of Claims 1 to 3 for the preparation of Spinosyn derivatives.

## Revendications

1. Composés de formule (I) 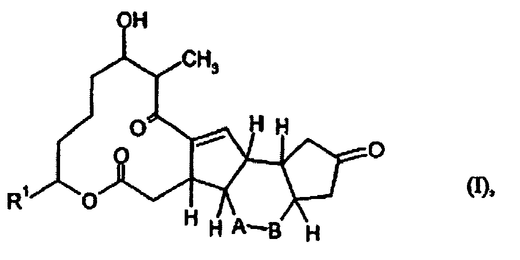 dans laquelle
R¹ représente un reste méthyle ou éthyle, et
A-B représente l'un des groupes suivants : -HC=CH-, -HC=C(CH₃)-, -H₂C-CH₂-, -H₂C-CH(CH₃)-.

2. Composés suivant la revendication 1, **caractérisés en ce que**
R¹ est un reste éthyle.

3. Composés suivant la revendication 1 ou 2, **caractérisés en ce que**
A-B représente le groupe -HC=CH-.

4. Procédé de production de composés suivant l'une des revendications 1 à 3, **caractérisé en ce qu'**on fait réagir des composés de formule générale (II) 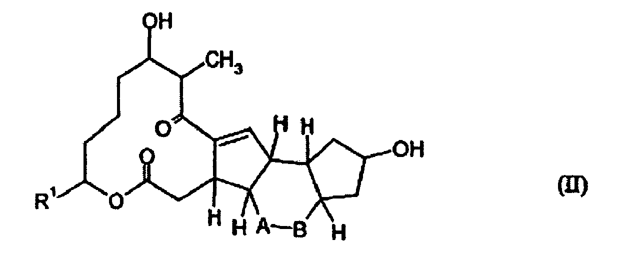 dans laquelle
R¹ et A-B ont les définitions indiquées dans l'une des revendications 1 à 3,
avec un agent oxydant, éventuellement en présence d'un diluant.

5. Procédé suivant la revendication 4, **caractérisé en ce qu'**on utilise comme agent oxydant le bichromate de pyridinium ou des N-halogénosuccinimides en présence de sulfure de diméthyle.

6. Procédé suivant la revendication 4 ou 5, **caractérisé en ce qu'**on utilise comme diluant le dichlorométhane.

7. Procédé de production de composés suivant l'une des revendications 1 à 3, **caractérisé en ce qu'**on met en contact des composés de formule générale (II) 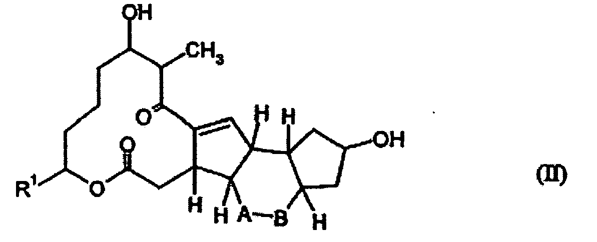 dans laquelle
R¹ et A-B ont les définitions indiquées dans l'une des revendications 1 à 3,
avec un micro-organisme dans un milieu nutritif aqueux dans des conditions aérobies ou avec un extrait d'enzyme préparé à partir de ce micro-organisme ou avec une ou plusieurs enzymes qui en sont isolées.

8. Procédé suivant la revendication 7, **caractérisé en ce qu'**on utilise comme micro-organisme un micro-organisme du genre Bacillus, du groupe des actinomycètes ou de la classe des zygomycètes.

9. Procédé suivant la revendication 8, **caractérisé en ce qu'**on utilise une souche de l'espèce Bacillus megaterium ou de l'espèce Bacillus simplex.

10. Procédé suivant la revendication 9, **caractérisé en ce qu'**on utilise la souche possèdant les caractères distinctifs des souches DSM 339 de Bacillus megaterium, DSM 333 de Bacillus megaterium ou DSM 14028 de Bacillus simplex.

11. Procédé suivant la revendication 8, **caractérisé en ce qu'**on utilise comme micro-organisme du groupe des actinomycètes un micro-organisme du genre Streptomyces.

12. Procédé suivant la revendication 11, **caractérisé en ce qu'**on utilise comme micro-organisme du genre Streptomyces une souche de l'espèce Streptomyces argillaceus, Streptomyces scabies, Streptomyces mirabilis ou Streptomyces pseudovenecuelae.

13. Procédé suivant la revendication 12, **caractérisé en ce que** la souche a les caractères distinctifs des souches DSM 14030 de Streptomyces argillaceus, DSM 14029 de Streptomyces scabies, DSM 14078 de Streptomyces mirabilis ou DSM 14079 de Streptomyces pseudovenecuelae.

14. Procédé suivant la revendication 8, **caractérisé en ce qu'**on utilise comme micro-organisme de la classe des zygomycètes un micro-organisme du genre Zygorhynchus, en particulier une souche de l'espèce Zygorhynchus moelleri.

15. Procédé suivant la revendication 14, **caractérisé en ce que** la souche présente les caractères distinctifs de la souche DSM 14198 de Zygorhynchus moelleri.

16. Procédé suivant la revendication 8, **caractérisé en ce qu'**on utilise comme micro-organisme de la classe des zygomycètes un micro-organisme du genre Mucor, en particulier une souche de l'espèce Mucor circinelloides.

17. Procédé suivant la revendication 16, **caractérisé en ce que** la souche a les caractères distinctifs de la souche DSM 14199 de Mucor circinelloides.

18. Procédé suivant l'une des revendications 4 à 17, **caractérisé en ce qu'**on isole les composés selon l'une des revendications 1 à 3.

19. Utilisation de composés selon l'une des revendications 1 à 3 pour la préparation de dérivés de spinosyne.
